# EUROPEAN PATENT SPECIFICATION

(11) **EP 1 465 475 B1**
(45) Date of publication and mention of the grant of the patent: **04.10.2006**
(21) Application number: 03717905.8
(22) Date of filing: 18.02.2003
(51) Int. Cl.: A01H 1/04, A01H 4/00, A01H 5/00, A01H 5/10, C12N 5/04

(54) **OROBANCHE RESISTANT SUNFLOWERS**
OROBANCHE-RESISTENTE SONNENBLUMEN
TOURNESOLS RéSISTANTES à L'OROBANCHE

(30) Priority: 20.02.2002 US 358206 P
(43) Date of publication of application: 13.10.2004
(73) Proprietor: PIONEER OVERSEAS CORPORATION, Des Moines, IA 50309 (US)
(72) Inventor: HASSAN, El Sayed, Abdel, Raouf, Sadik, El Mansoura (EG)
(74) Representative: Bentham, Andrew
(86) International application number: PCT/US2003/004931
(87) International publication number: WO 2003/073837

(56) References cited:
- FERNANDEZ-MARTINEZ J ET AL: "Selection of wild and cultivated sunflower for resistance to a new broomrape race that overcomes resistance of the Or5 gene" CROP SCIENCE, vol. 40, no. 2, May 2000 (2000-05), pages 550-555, XP008038438 ISSN: 0011-183X
- SUKNO S ET AL: "Reproductive behaviour and broomrape resistance in interspecific hybrids of sunflower" PLANT BREEDING, vol. 117, no. 3, July 1998 (1998-07), pages 279-285, XP008038448 ISSN: 0179-9541
- POGORLETSKII B K ET AL: "IMMUNITY OF SUNFLOWER TO BROOMRAPE" GENETIKA, vol. 11, no. 7, 1975, pages 18-26, XP008038444 ISSN: 0016-6758
- SUKNO S ET AL: "Inheritance of resistance to Orobanche cernua Loefl. in six sunflower lines" CROP SCIENCE, vol. 39, no. 3, May 1999 (1999-05), pages 674-678, XP008038440 ISSN: 0011-183X
- MELERO-VARA J M ET AL: "EVALUATION OF DIFFERENTIAL LINES AND A COLLECTION OF SUNFLOWER PARENTAL LINES FOR RESISTANCE TO BROOMRAPE OROBANCHE-CERNUA IN SPAIN" PLANT BREEDING, vol. 102, no. 4, 1989, pages 322-326, XP008038451 ISSN: 0179-9541
- LABROUSSE P. ET AL.: 'Several mechanisms are involved in resistance of helianthus to orobanche cumana wallr' ANNALS OF BOTANY vol. 88, 2001, pages 859 - 868, XP008037223

## Description

### Field of the Invention

This invention relates to methods of producing sunflower plants, plant parts, and seeds that produce sunflower plants having resistance to *Orobanche cumana.*

### Industrial Applicability

Cultivated sunflower (*Helianthus annuus* L.) is being grown as an increasingly important oilseed crop in many temperate, semi-dry regions of the world.

The cultivated sunflower is a major worldwide source of vegetable oil. Oil types of sunflowers contain 40 to 48 percent oil in the seed. Sunflower oil is valued as an edible oil because of its high unsaturated fat level and light color. Sunflower oil is used for salads, cooking oil or for margarine. The protein content of sunflower meal prepared from seeds after oil extraction is useful as livestock feed. The seeds from both oil and confectionery varieties of cultivated sunflower are useful as bird food.

The parasitic plant *Orobanche spp.* or broomrape has become a limiting factor for sunflower crops in infested countries. The decrease in crop yield can reach 95% in an affected field. Particular *Orobanche* species afflicting sunflower include *Orobanche aegyptiaca* Pers., *O. ramosa* L., *O. minor* Sm., *O. cumana* Wallr. and *O. cernua* Loefl.

*Orobanche, cumana* Wallr. and *O. cernua* Loefl. (alternative names for the same species) is a severe pest in sunflower in eastern Europe and has been spreading through southern Europe. Over the past few years the progression of this parasitic plant, its introduction into new countries, and the development of new and more virulent races have all been observed. *Orobanche* presents a worldwide risk, and some species such as *O. minor* have appeared as exotics in the United States.

Starting in 1996, the emergence of 3 new races of broomrape were observed in fields in Turkey, Spain and Bulgaria. By 2000, the number of the affected fields in these areas had sharply increased. Some producers had stopped growing sunflower because of the significant reduction in grain yield experienced.

These weeds are obligate root holoparasites. *Orobanche* species are very difficult to eliminate because except for their flower parts, they live in the soil; their seeds are minute and prolifically produced, easily dispersed and very long-lived. Thus, herbicides presently used in sunflower generally provide inadequate control.

Other means of controlling *Orobanche* include biological control agents, isolation of the genes responsible for *Orobanche* resistance in sunflower, and development of resistant sunflower lines.

It has been reported that *Fusarium oxysporum* f. sp. *orthoceras* may be a potential agent for the biological control of *Orobanche cumana* (Thomas-Heiko et al., *Biological Control.,* Sept., 1998; 13(1):41-48). However application methods and uniform doses under field conditions have yet to be determined.

The Or3 gene confers resistance to *Orobanche* attack but is known to be efficacious only against race C. Up to five race variants of *Orobanche* have been reported with anecdotal evidence of several more. (Antonova, T.S. et al, *Weed Research.,* 1996, 36(2):113-121).

Identification of additional sources of resistance to *Orobanche,* especially to new races, is beneficial in managing race shifts and production loss.

### Summary of the Invention

This invention relates to compositions and methods for producing sunflower plants and sunflower lines resistant to new races of *Orobanche cumana,* the method comprising crossing a first parent sunflower with a second parent sunflower and harvesting the resultant seed, wherein the first sunflower parent is the sunflower line represented by ATCC deposit number PTA-3793.

Accordingly, the invention provides the following.

A sunflower plant resistant to *Orobanche cumana* races in addition to races A-E, said plant being obtainable by a method comprising:
crossing a first parent sunflower plant to a second parent sunflower plant and harvesting the resultant progeny seed, wherein the first sunflower plant is a plant grown from the seeds of line U00S9LM, represented by ATCC Accession #PTA-3793.

A plant part of the plant of the invention.

A cell of a plant of the invention.

A tissue culture of regenerable cells of the invention,

A method of regenerating a sunflower plant of the invention, comprising growing said tissue culture of claim under conditions sufficient for regenerating said sunflower plant.

A method for preparing oil and/or meal of seed of a sunflower plant, the method comprising growing a plant of the invention, harvesting seed therefrom, crushing said seed and separating the oil and/or meal.

### Detailed Description

The present invention pertains to compositions and methods for producing sunflower plants and sunflower lines having highly heritable *Orobanche* resistance traits obtained through breeding and selection. These lines are useful in developing commercial cultivars of sunflower crops having other valuable agronomic and grain and/or seed quality traits.

Unless otherwise defined, all technical and scientific terms used herein have the same meaning as commonly understood by one of ordinary skill in the art to which this invention belongs. Unless mention ed otherwise, the techniques employed or contemplated herein are standard methodologies well known to one of skill in the art.

In the context of this disclosure, a number of terms will be used. In order to provide a clear and consistent understanding of the specification and claims, including the scope to be given such terms, the following definitions are provided.

"Broomrape", "*Orobanche cumana*" "*Orobanche cumana* Wallr.", "*O. cemua* Loefl" and "*O. cumana*" are herein used interchangebly to refer to the described parasitic weed and it's races.

"Crossing" (or "cross-pollination") refers to the transfer of pollen from one plant to a different plant.

"Cytoplasmic Male Sterile" or "CMS": A sunflower line that produces no viable pollen is called male sterile. Cytoplasmic male sterility is inherited maternally, i.e. the male sterile plant is used as the female parent in a cross with pollen from another sunflower. CMS lines are produced by crossing a maintainer line with a sunflower plant with the cytoplasmic male sterility trait and then backcrossing to the maintainer line until a male sterile line that is homologous to the maintainer line in all other respects is developed. CMS lines are also referred to as female lines.

"Line" means a group of plants that display less variation between individuals, generally as a result of several generations of self-pollination. Also, a line can include a group of plants vegetatively propagated from a single parent plant, using tissue or cell culture technique.

"Plant or plant parts" refer to plant cells, plant protoplasts, plant cell tissue cultures from which sunflower plants can be regenerated, plant calli, plant clumps, and plant cells that are intact in plants or parts of plants such as embryos, pollen, ovules, flowers, leaves, husks, stalks, roots, root tips, anthers, seed and seed meal, and the like.

For the purpose of this invention, "broomrape (or *Orobanche*) resistance" is defined as a genetically controlled attribute of sunflower plants that prevents a parasitic broomrape plant from attaching to a sunflower plant and completing its life cycle through the reproductive (seed-bearing) stage. The resistance may result from several plant-parasite responses, including but not necessarily limited to : (1) lack of sufficient broomrape seed germination stimulus from the sunflower plant (assumed to be a chemical root exudate); (2) blockage of broomrape haustorial penetration of the sunflower root surface or (3) blockage of broomrape haustorial connection to the vascular system of the sunflower plant.

"New" races of *Orobanche cumana* is defined as races beyond the currently characterized races A-E.

"Restorer line": line possessing the gene or genes to restore male fertility or viable pollen to a sunflower hybrid or inbred line and progeny having a maternal cytoplasm that conditions male sterility. This term is also discussed in the literature. See for e.g. Fick, "Breeding and Genetics," in Sunflower Science and Technology 279-338 (J.F. Carter ed. 1978).

"Selection", means the extraction of a desired phenotype from a broad, genetically heterogeneous breeding pool, commonly termed a population. Individual plants within the population are selected for traits associated with plant morphology, flower morphology, insect and disease resistance, maturity and yield.

"Variety" or "cultivar' refers to a group of plants within the species (e.g., Helianthus annuus) which share certain constant characteristics that separate them from the typical form and from other possible varieties within that species. While possessing at least one distinctive trait, a 'variety' is also characterized by a substantial amount of overall variation between individuals within the variety, based primarily upon the Mendelian segregation of traits among the progeny of succeeding generations.

Field crops are bred through techniques that take advantage of the plant's method of pollination. A plant is self-pollinated if pollen from one flower is transferred to the same or another flower of the same plant. A plant is cross-pollinated if the pollen comes from a flower on a different plant. Thus the terms "selfed" in a breeding program refers to self-pollination and the term "crossed" means a plant pollinated from a different plant.

Sunflowers have a head-type inflorescence with sterile ray flowers and perfect (having both stamen and pistil) disc flowers. In it's wild state, the sunflower has been self sterile or self-incompatible so it is a highly cross-pollinating crop that depends largely on honeybees for pollination. Most production areas do not have a sufficient bee population to adequately pollinate the crop. This has resulted in breeder selection for self-compatibility or self-fertility which increases the yield of seed when few bees are present.

A sunflower breeding population should be substantially homogenous and reproducible to be useful in either further breeding or the development of a commercial cultivar. There are a number of analytical methods available to determine the phenotypic stability of a sunflower population.

The oldest and most traditional method of analysis is the observation of phenotypic traits. The data is usually collected in field experiments over the life of the sunflower plants to be examined. Phenotypic characteristics most often are observed for traits associated with seed yield, seed oil content, seed protein content, fatty acid composition of oil, glucosinolate content of meal, growth habit, lodging resistance, plant height, shattering resistance, etc. Other phenotypic characteristics commonly observed include resistance to disease, insects and tolerance to herbicides.

It has been found that the compositions and methods of the present invention can create sunflower plants through the combination of sunflower genetic determinants that heretofore were not recognized to make possible the formation of novel lines of sunflowers resistant to new races of *Orobanche cumana.*

Several new races of *Orobanche* were identified in production fields in Turkey. A set of sunflower germplasm was field screened in Turkey the following season. Two types of resistance responses were identified in wild species sources crossed to Pioneer germplasm. Both provided resistance to the new races.

One source of resistance (System 1) appears to act by preventing the penetration of haustorium from *Orobanche* into the root tissue of the host. This form of resistance is represented in sunflower line U00S9LM and ATCC Accession # PTA-3793. This is the resistance source of the invention as claimed herein.

A second source of resistance (System 2) appears to act by inhibiting the development of parasitic shoots that have established on the root system of the host but before emerging above the soil level. This form of resistance is represented in sunflower line 8556UG and ATCC Accession # PTA-3792.

A third mechanism combines the sources of resistance of both systems to create a stronger resistance. This form of resistance is represented in sunflower line 01 UL2365 and ATCC Accession #PTA-3791.

The compositions and methods of the present invention can reproducibly create sunflower lines that express the *O*. *cumana* (broomrape) resistance trait against phenotypic backgrounds of high seed yield, faster maturity, drought tolerance, cold tolerance, increased seed protein content, modification of composition of amino acid content in seeds, dwarfism, resistance to lodging, resistance to insects or diseases caused by bacteria, fungi, or viruses, or tolerance to herbicide treatment which is sufficiently consistent for commercial applications. In contrast, the original cultivars displayed extensive variability in the expression of such characteristics.

To be easily incorporated in breeding programs combining desirable traits, the trait for *Orobanche* resistance should be highly heritable, ie dominant or semi-dominant. This was found to be true in the presently claimed compositions and methods (see Example 3).

### Hybrid development in sunflower

Hybrid sunflower seed is typically produced by a male sterility system incorporating genetic or cytoplasmic male-sterile (CMS) inbreds. Plants of a CMS inbred are male sterile as a result of factors resulting from the cytoplasmic, as opposed to the nuclear, genome. Thus, this characteristic is inherited exclusively through the female parent in sunflower plants, since only the female provides cytoplasm to the fertilized seed. CMS plants are fertilized with pollen from another inbred that is not male-sterile. Pollen from the second inbred may or may not contribute genes that make the hybrid plants male-fertile.

Cytoplasmic male sterile lines are traditionally developed by the backcrossing method in which desirable lines that have undergone inbreeding and selection for several generations are crossed initially to a plant with cytoplasmic male sterility. Thereafter the inbred line to be converted is used as a recurrent parent in the backcrossing procedure. The final progeny will be genetically similar to the recurrent parent except that it will be male sterile.

The derived starting material of the present invention can be converted to cytoplasmic male sterility (CMS) by crossing the deposited seeds with a sunflower lines well known in the art and publicly available such as CMS HA89 (USDA), that incorporates a cytoplasmic determinant for male sterility. The source of CMS HA89 is from the material of Leclercq, "Cytoplasmic Sterility in the Sunflower," *Ann. Amelior Plant* 19:99-106 (1969).

Fertility restorer lines are developed by transferring a dominant restorer gene to an established inbred line with normal cytoplasm by backcrossing. If this procedure is used, selected plants must be crossed to a cytoplasmic male sterile line after each generation to determine if the fertility restorer genes are present. A more common procedure is self-pollination and selection of male fertile plants from commercial hybrids or planned crosses of parents having restorer genes in male sterile cytoplasm. This procedure does not require test crossing to a male sterile line during selection because the plants will be fully male fertile if the necessary restoring genes are present.

A suitable restorer line can be produced by crossing the germplasm of the present invention with any of the commonly available restorer lines, such as RHA274, RHA271, and RHA273 (USDA) or other lines possessing genes for restoration of male fertility. Lines and varieties thus derived which produce seed having broomrape resistance and which are true-breeding for at least the fertility restorer genes, can then be isolated by continuous self-pollination and crossed with the broomrape resistant CMS lines previously described.

This invention is also directed to methods for producing an F1 hybrid seed by crossing a first parent sunflower plant with a second parent sunflower plant, wherein the first parent plant is grown from the seeds of line U00S9LM, represented by ATCC Accession # PTA-3793.

This invention is also related to the cells and other parts of those plants.

### Inbred development in sunflower

The use of male sterile inbreds is but one factor in the production of sunflower hybrids. The development of sunflower hybrids requires, in general, the development of homozygous inbred lines, the crossing of these lines, and the evaluation of the crosses. Pedigree breeding and recurrent selection breeding methods are used to develop inbred lines from breeding populations. Breeding programs combine the genetic backgrounds from two or more inbred lines or various other broad-based sources into breeding pools from which new inbred lines are developed by selfing and selection of desired phenotypes. The new inbreds are crossed with other inbred lines and the hybrids from these crosses are evaluated to determine which of those have commercial potential.

Pedigree breeding is commonly used for the improvement crops. Pedigree breeding starts with the crossing of two genotypes, each of which may have one or more desirable characteristics that is lacking in the other or which complements the other. If the two original parents do not provide all of the desired characteristics; additional parents can be included in the crossing scheme.

These parents are crossed in a simple or complex manner to produce an F1. An F2 population is produced by selfing one or several F1's or by intercrossing two F1's (i.e., sib mating). Selection of the best individuals may begin in the F2 population, and beginning in the F3, the best families, and the best individuals within the best families, are selected. Replicated testing of families (lines) can begin in the F4 generation to improve the effectiveness of selection for traits with low heritability. At an advanced stage of inbreeding (i.e., F6 and F7), the best lines or mixtures of phenotypically similar lines commonly are tested for potential release as new cultivars.

Improved varieties may also be developed through recurrent selection. A genetically variable population of heterozygous individuals is either identified or created by intercrossing several different parents. The best plants are selected based on individual superiority, outstanding progeny, or excellent combining ability. The selected plants are intercrossed to produce a new population in which further cycles of selection are continued.

The objective of commercial sunflower hybrid line development programs is to develop new inbred lines to produce hybrids that combine to produce high yields and superior agronomic performance. The primary trait breeders seek is yield. However, many other major agronomic traits are of importance in hybrid combination and have an impact on yield or otherwise provide superior performance in hybrid combinations. Major objectives in sunflower breeding include improved seed yield, improved seed oil percentage or oil quality, earlier maturity, shorter plant height, uniformity of plant type, or disease or insect resistance. In addition, the lines per se preferably have acceptable performance for parental traits such as seed yields and pollen production, all of which affect ability to provide parental lines in sufficient quantity and quality for hybridization. These traits have been shown to be under genetic control and many if not all of the traits are affected by multiple genes.

Although sterility techniques, including the use of CMS determinants, may be advantageously applied in producing broomrape resistant parent lines and varieties as well as hybrids within the present invention, the application of such techniques is not a prerequisite to practice the invention. Parent lines and varieties using the methods of the invention can be produced by manipulation of existing sunflower materials, using other conventional methods, based on successive selections and inbreeding, or newly developed molecular approaches to altering the genetic content of plants. In any event, the production of suitable parent lines and varieties in accordance with the present invention entails the elimination of a certain amount of variability, at least to the extent that an appreciable number of progeny derived from self-pollinating at least one of the parents produce seed having broomrape resistant traits.

Through breeding techniques, improved resistance to *Orobanche cumana* can be combined or "stacked" with any other desirable seed, agronomic, or insect- or disease-resistance trait. Examples of other desirable traits include, but are not limited to altered seed oil profile or content, high seed yield, faster maturity, drought tolerance, cold tolerance, increased seed protein content, modification of composition of amino acid content in seeds, dwarfism, resistance to lodging, resistance to insects or diseases caused by bacteria, fungi, or viruses, or tolerance to herbicide treatment.

The methods of the present invention find particular use in breeding programs with sunflower lines tolerant to sulfonylurea herbicide. Sulfonylurea herbicides have been found effective against parasitic weeds of sunflower.such as dodder and *Orobanche* species (L. Garcia-Torres et al. *Weed Research,* 1994, 34:395-402).

### Regeneration of plants

This invention also relates to the parts of the plants of the invention, including plant cells, plant protoplasts, plant cell tissue cultures from which sunflower plants can be regenerated, plant calli, plant clumps, and plant cells that are intact in plants or parts of plants such as embryos, pollen, ovules, flowers, leaves, husks, stalks, roots, root tips, anthers, seed and seed meal, and the like.

The plants produced in accordance with the present invention may be regenerated from plant parts using known techniques. For instance, seeds from the plants of the present invention may be planted in accordance with conventional sunflower growing procedures. These plants will generate further seeds following self-pollination.

Sunflower plants may also be regenerated using tissue culture and regeneration. Culture of various cells and tissues of sunflower and regeneration of plants therefrom is known to those skilled in the art. For example; the propagation of sunflower by tissue culture is described in the following references: Henderson *et al,* "The culture of normal sunflower stem callus." (1952), *Am. J. Bot.* 39:444-451; Levine, M., "Response of fibrous roots of sunflower and tobacco tissue cultures to plant growth substances" (1951), *Bot. Gaz.* 112:281-289; Henrickson, C.E., "The flowering of sunflower explants in aseptic culture" (1954), *Plant Physiol.* 29:536-538; and Sadhu, M. K., "Effect of different auxins on growth and differentiation in callus tissue from sunflower stem pith" (1974), *Indian J. Exp. Biol.* 12:110-111.

### Vegetable Oil and Meal

The seed of the plants of this invention may be used for producing vegetable oil and meal. The seed of these varieties, the plant produced from such seed, the hybrid sunflower plant produced from the crossing of these varieties with other varieties, the resulting hybrid seed, and various parts of the hybrid sunflower plant can be utilized in the production of an edible vegetable oil or other food products in accordance with known techniques.

Sunflower kemels are encased in a protective hull which creates the need for dehulling prior to oil extraction and further processing. Most modem plants specifically designed to crush sunflowers have dehulling equipment although the seed can be crushed less efficiently in its entirety with hulls still on. Most U.S. sunflower processing plants use a combination screw press and solvent extraction to extract the oil. It is reported to be a very efficient method of removing oil from the seed, leaving all but about 1% of the oil in the remaining plant material.

Crude sunflower oil is further processed for use in food products. Refining reduces the free fatty acid content and removes impurities. The bleaching process ensures problem-causing pigments are removed. Winterizing involves the gradual chilling of the oil so that as a salad oil it can withstand storage under refrigeration without clouding. Deodorizing removes remaining impurities to improve oil flavor and odor. When an edible oil is described as RBD, it has undergone the processing stages of refining, bleaching and deodorizing.

The remaining solid meal component derived from seeds can be used as a nutritious livestock feed. Sunflower meal is a high protein by-product of sunflower oil processing. It is used primarily as a livestock feed additive to supplement protein levels and readily substitutes for soybean meal. There are no toxic factors and very low levels of anti-nutrient factors (such as trypsin inhibitors) which require additional processing. It is the fourth largest source of oilseed meal. The protein quality of sunflower seed meal is high: it has an essential amino acid index of 68, which compares favorably with soybean meal and is limiting only in lysine.

### Examples

### Notes:

1) The summer nursery location was Ahmetbey, Luleburgaz, Kirklarli, Turkey. Tests at this nursery were under conditions of heavy, artificial infestation with seeds of the new race(s) of *Orobanche cumana* collected from naturally, heavily infested farm fields.
2) "Heavy infestation" or "heavily infested" is defined as an infestation of the new race(s) of O. *cumana* sufficient to reduce yields by 80-100% in susceptible sunflower lines.
3) The winter nursery location was at Kaha, Kalyobia, Egypt, in fields completely free of O. *cumana* infestation.
4) Naturally and heavily infested fields were located at Tekke koy, Malkara, Tekirdag, Turkey.
5) Resistance to broomrape was assessed (in field screenings) just prior to harvesting by counting the number of sunflower plants that were free from infection with broomrape within a radius of 50 cm around each plant.
6) Susceptible individuals had many emerged and/or attached broomrape shoots. No intermediate types of resistance were found. The susceptible plants showed differing degrees of attack intensity.

### Example 1:

### Screening for System 1 resistance in the growth chambers

165 samples of sunflower seeds (165 individual heads) with suspected resistance to the new races of O. *cumana* (broomrape) were gathered from infested fields at Thrace, Turkey. The seeds were wild outcrosses of Pioneer proprietary germplasm developed in Woodland, CA.

The sunflower seeds were planted in 5" pots in a mixture of sand and soil (1:1) that were infested with seeds of the new races of O. *cumana* at the rate of 100 grams/1kg. The sunflower seeds were planted at a rate of ten sunflower seeds/pot. Plants were grown in a growth chamber at 25°C under continuous light. NPK-containing fertilizer was added once during the growing period. After 75 days from sowing, the plants were removed from the pots, the root systems washed, and the number of resistant plants per pot recorded. The remaining seeds of those samples that were shown to segregate in the pot tests, were used in field screening tests.

### Example 2:

### Selection for plants with System 1 resistance

As a result of the growth chamber screening, seeds of the heads that showed segregation for resistance were planted as F1 seed in the following summer nursery. Seeds of the new races of *O. cumana* that were collected from naturally infested sunflower fields were used to artificially infest the test plots. The resistant F1 plants were harvested and their seeds planted as F2's in the winter nursery.

### a) Hybrid combinations:

From these F2 progenies, 20 heads were selected for putative resistance and individual crosses were made head to head with 4 different susceptible female inbred lines to create test cross hybrids:

Screening of the test cross and new hybrids for the resistance to the new races of broomrape were conducted for several years in Tekke koy, Malkara, Tekirdag, Turkey, in fields naturally and heavily infested with the newly observed races of broomrape. These were fields in which the previous yield of sunflower crops were reduced by approximately 80-100% due to the attack of the new races of broomrape. Screenings were performed using a randomized block design with 2 replications per each hybrid cross. Resistance to broomrape was assessed just prior to harvesting by counting the number of sunflower plants that were free of infection with broomrape within a radius of 50 cm around each plant. Resistant plants were removed from the ground to confirm the absence of any broomrape shoots that may be attached to the root system below the soil surface.

Selection for resistance and test-crossing continued in heavily, naturally infested fields, until the F7 generation. At this point it was determined that the F7 lines were fixed for resistance, fertility, consistent oil content, and lack of seed shattering.

### b) Inbred lines and breeding germplasm:

From the F2 head selections, F3 seeds were planted in the summer nursery.

Screening of the new source of breeding germplasm and inbred lines for the resistance to the new races of broomrape were conducted over several years in Ahmetbey, Luleburgaz-Kirklarli, Turkey. The summer screening nursery was artificially infested with seeds of the new races of *O. cumana* that were collected from naturally heavily infested sunflower fields. The soil infestation of the nursery was repeated every year. Screenings were performed by planting 15 plants in 4 meter row lengths for each selection of the breeding germplasm and inbred lines. Resistance to broomrape was assessed just prior to harvesting by counting the number of sunflower plants that were free of infection with broomrape within a radius of 50 cm around each plant. Putative resistant plants were removed from the ground to confirm the absence of any broomrape shoots that might be attached to the root system below the soil surface.

This line is represented by ATCC deposit number PTA-3793.

### Example 3

### Inheritance study of System 1

Crosses were made between resistant and susceptible Pioneer inbred lines to create two populations for this inheritance study. These two populations were as follows:
a) U9605LF (susceptible)/U00T3LM (resistant).
b) U9612LM (susceptible)/U00ZOLM (resistant).

The F2 population of U9605LF/U00T3LM and its F1 backcross were planted in a field heavily and naturally infested with the seeds of the new races of broomrape. Five replications of 250 plants each were planted in Tekke koy, Malkara, Tekirdag, Turkey.

The F2 population of U9612LM/U00ZOLM was planted in the same field in Turkey in 4 replications (250 plants each) and another 526 plants of the same population were planted in naturally infested field in La Carlota, Cordoba, Spain.

As shown in the table below, the F2 plants of the two populations segregated 3:1 resistant-to-susceptible, while the plants of the F1 backcross segregated 1:1 resistant-to- susceptible, demonstrating that the resistance to the new races via System 1 is controlled by a single dominant gene.

### Data collected from the inheritance study:

### I. F2 population of U9505LF/U00T3LM and its F1BC1

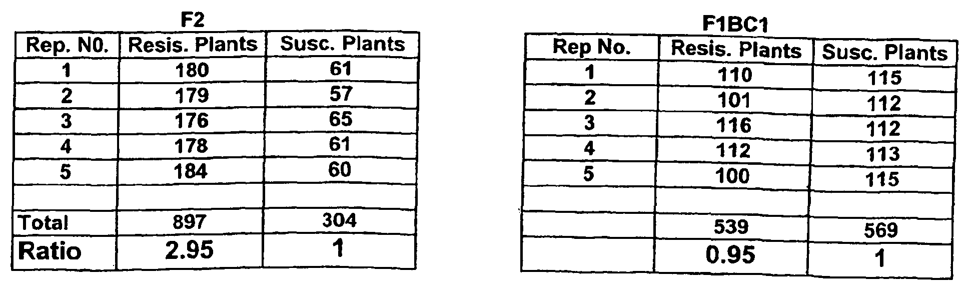

### II. F2 mapping population of U9612LM/ UOOZOLM

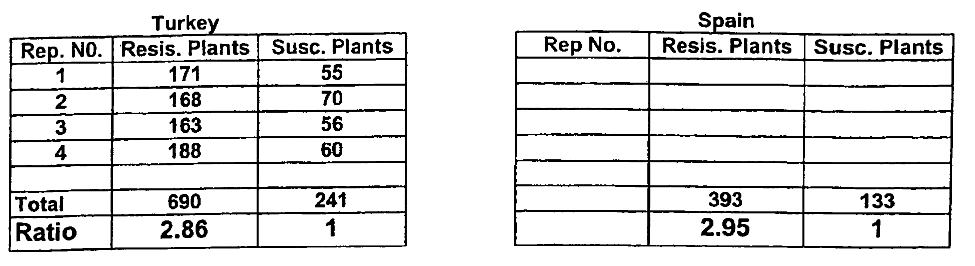

### Example 4:

### Selection and Field Screening for Plants with System 2 resistance

System 2 resistance was found within the germplasm of the Freeman Johnson collection (purchased by Pioneer). This source was utilized for breeding with Pioneer's key maintainer inbred lines.

Materials were received from Woodland, CA. They were planted as F3 seed in the summer nursery (Ahmetbey, Luleburgaz, Kirklareli, Turkey). Resistant plants were selected, harvested, and their seeds planted in the winter nursery. From the F4 plants, about 20 heads were selected and crossed head to head with U9605LF (a susceptible female inbred line) for making test cross hybrids.

### a) Hybrid combinations:

Screening of the test crosses and new hybrids for System 2 resistance to the new races of broomrape were conducted over several years in Tekke koy, Malkara, Tekirdag, Turkey in fields naturally and heavily infested with the newly observed races of broomrape. These were fields in which the previous yield of sunflower crops were reduced by approximately 80-100% due to the severe attack of the new races of broomrape. Screenings were performed using a randomized block design with 2 replications per each hybrid cross. Resistance to broomrape was assessed just prior to harvesting by pulling the plants and counting the number of sunflower plants which had dead broomrape shoots attached on its root system under the ground.

Susceptible individuals had many emerged broomrape shoots and showed different degrees of attack intensity.

### b) Inbred lines and breeding germplasm:

Screening of the new source of breeding germplasm and inbred lines for the resistance to the new races of broomrape were conducted over several years in the summer nursery at Ahmetbey, Luleburgaz-Kirklarli, Turkey. The nursery was artificially infested with seeds of the new races of *O. cumana.* The soil infestation of the nursery was repeated every year. Screening was performed by planting 15 plants in a 4 meter row length for each selection of the breeding germplasm and inbred lines. Resistance to broomrape was assessed just prior to harvesting by removing plants from the ground and counting the number of sunflower plants that had dead broomrape shoots attached to its root system below the soil surface.

Susceptible individuals had many emerged broomrape shoots. The susceptible plants were showing different degrees of broomrape attack intensity.

### Example 5:

### Field Screening and Selection for Hybrid Plants with the combination of System 1 and System 2 resistance

A hybrid combination of System 1 and System 2 were made to strengthen the resistance against any future emerging races of broomrape. The cross (01 Ul2365, represented by ATCC deposit number PTA-3791) was made between U0170LF (System 2, female) and U00S9LM (System 1, male). The hybrid cross was screened at Tekke koy, Malkara, Tekirdag, Turkey in fields naturally and heavily infested with the newly observed races of broomrape. These were fields in which the previous yield of sunflower crops were reduced by approximately 80-100% due to the severe attack of the new races of broomrape. Screenings were performed using a randomized block design with 4 replications. Resistance to broomrape was assessed just prior to harvesting by counting the number of sunflower plants which were free of infection with broomrape within a radius of 50 cm around each plant and also by pulling the plants and counting the number of sunflower plants which had dead broomrape shoots attached on its root system under the ground. No susceptible plants of this hybrid combination were observed. About 1 to 3 % of these resistant plants had attached dead broomrape shoots under the ground, confirming expression of System 2 resistance. The plants of the susceptible controls died before the flowering stage due to severe attack by the new races of broomrape.

## Claims

1. A sunflower plant resistant to *Orobanche cumana* races in addition to races A-E, said plant being obtainable by a method comprising:
crossing a first parent sunflower plant to a second parent sunflower plant and harvesting the resultant progeny seed, wherein the first sunflower plant is a plant grown from the seeds of line U00S9LM, represented by ATCC Accession #PTA-3793.

2. A plant part of the plant of claim 1.

3. A cell of a plant of claim 1.

4. A tissue culture of regenerable cells of claim 3.

5. A method of regenerating a sunflower plant of claim 1, comprising growing the tissue culture of claim 4 under conditions sufficient for regenerating said sunflower plant.

6. A method for preparing oil and/or meal of seed of a sunflower plant, the method comprising growing a plant of claim 1, harvesting seed therefrom, crushing said seed and separating the oil and/or meal.

7. A method of claim 6 further comprising further processing said oil for use in food products.

8. A method of claim 7 wherein said further processing comprises refining, bleaching, winterising or deodorizing.

9. A method of claim 8 wherein said further processing involves refining, bleaching and deodorizing.

10. A method of claim 9 further comprising feeding said meal to livestock.

## Patentansprüche

1. Sonnenblumenpflanze, die gegen *Orobanche-cumana*-Rassen, zusätzlich zu den Rassen A-E, resistent ist, wobei die Pflanze durch ein Verfahren erhalten werden kann, das umfasst:
Kreuzen einer ersten Eltern-Sonnenblumenpflanze mit einer zweiten Eltern-Sonnenblumenpflanze und Ernten der resultierenden Nachkommensamen, wobei die erste Sonnenblumenpflanze eine Pflanze ist, die aus den Samen der Linie U00S9LM, wiedergegeben durch ATCC-Eingangsnummer PTA-3793, gezogen worden ist.

2. Pflanzenteil der Pflanze nach Anspruch 1.

3. Zelle einer Pflanze nach Anspruch 1.

4. Gewebskultur von regenerierbaren Zellen nach Anspruch 3.

5. Verfahren zum Regenerieren einer Sonnenblumenpflanze nach Anspruch 1, das Züchten der Gewebskultur nach Anspruch 4 unter Bedingungen, die zum Regenerieren der Sonnenblumenpflanze hinreichend sind, umfasst.

6. Verfahren zum Herstellen von Öl und/oder Mehl aus Samen einer Sonnenblumenpflanze, wobei das Verfahren Züchten einer Pflanze nach Anspruch 1, Ernten von Samen davon, Schroten der Samen und Abtrennen des Öls und/oder Mehls umfasst.

7. Verfahren nach Anspruch 6, das weiterhin weitere Bearbeitung des Öls zur Verwendung in Nahrungsmitteln umfasst.

8. Verfahren nach Anspruch 7, wobei die weitere Bearbeitung Raffination, Bleichen, Winterisieren oder Desodorierung umfasst.

9. Verfahren nach Anspruch 8, wobei die weitere Bearbeitung Raffination, Bleichen und Desodorierung umfasst.

10. Verfahren nach Anspruch 9, das weiterhin Verfüttern des Schrots an Vieh umfasst.

## Revendications

1. Plante de tournesol résistante à des races d'*Orobanche cumana,* en plus des races A-E, ladite plante pouvant être obtenue par un procédé comprenant :
le croisement d'une première plante de tournesol parent avec une seconde plante de tournesol parent et la récolte des graines de la descendance obtenues, dans lequel ladite première plante de tournesol est une plante cultivée à partir de graines de la lignée U00S9LM, représentée par le numéro d'accès ATCC PTA-3793.

2. Partie de plante de la revendication 1.

3. Cellule d'une plante de la revendication 1.

4. Culture de tissu de cellules pouvant être régénérées de la revendication 3.

5. Procédé de régénération d'une plante de tournesol de la revendication, comprenant la croissance de la culture de tissu de la revendication 4 dans des conditions suffisantes pour régénérer ladite plante de tournesol.

6. Procédé pour préparer de l'huile et/ou de la farine de graines d'une plante de tournesol, le procédé comprenant la culture d'une plante de la revendication 1, la récolte de ses graines, le broyage desdites graines et la séparation de l'huile et/ou de la farine.

7. Procédé de la revendication 6, comprenant en outre la transformation additionnelle de ladite huile pour une utilisation dans des produits alimentaires.

8. Procédé de la revendication 7, dans lequel ladite transformation additionnelle comprend un raffinage, une décoloration, une démargarination ou une désodorisation.

9. Procédé de la revendication 8, dans lequel ladite transformation additionnelle comprend un raffinage, une décoloration, et une désodorisation.

10. Procédé de la revendication 9, comprenant en outre l'alimentation d'animaux d'élevage par ladite farine.
